# EUROPEAN PATENT SPECIFICATION

(11) **EP 3 266 771 B1**
(45) Date of publication and mention of the grant of the patent: **20.02.2019**
(21) Application number: 16174002.2
(22) Date of filing: 10.06.2016
(51) Int. Cl.: C07D 311/18, C07C 217/54, C07C 213/00, C07C 201/12, C07C 205/37, C07C 37/20, C07C 39/23

(54) **SYNTHESIS OF PHOTOACTIVABLE CAGED CYCLOFEN-OH AND DERIVATIVES THEREOF**
SYNTHESE VON LICHTAKTIVIERBAREM EINGEKAPSELTEM CYCLOFEN-OH UND DERIVATEN DAVON
SYNTHÈSE DE CYCLOFEN-OH EN CAGE PHOTOACTIVABLE ET SES DÉRIVÉS

(43) Date of publication of application: 10.01.2018
(73) Proprietor: PARIS SCIENCES ET LETTRES - QUARTIER LATIN, 75006 Paris (FR); CENTRE NATIONAL DE LA RECHERCHE SCIENTIFIQUE, 75016 Paris (FR); SORBONNE UNIVERSITE, 75006 Paris (FR)
(72) Inventor: JULLIEN, Ludovic, 94110 Arcueil (FR); AUJARD, Isabelle, 75012 Paris (FR)
(74) Representative: Icosa

(56) References cited:
- WO-A1-2008/030771
- DEEPAK KUMAR SINHA ET AL: "Photocontrol of Protein Activity in Cultured Cells and Zebrafish with One- and Two-Photon Illumination", CHEMBIOCHEM - A EUROPEAN JOURNAL OF CHEMICAL BIOLOGY., vol. 11, no. 5, 24 February 2010 (2010-02-24), pages 653-663, XP055313167, DE ISSN: 1439-4227, DOI: 10.1002/cbic.201000008
- LUDOVIC FOURNIER ET AL: "A Blue-Absorbing Photolabile Protecting Group for in Vivo Chromatically Orthogonal Photoactivation", ACS CHEMICAL BIOLOGY, vol. 8, no. 7, 19 July 2013 (2013-07-19), pages 1528-1536, XP055212890, ISSN: 1554-8929, DOI: 10.1021/cb400178m

## Description

### FIELD OF INVENTION

The present invention relates to a new process of manufacturing of photoactivable caged precursors, which are useful for understanding the spatiotemporal dynamics of biological cells. Especially, the invention relates to a process of manufacturing of caged cyclofen-OH and derivatives thereof, more specifically of compounds of formula (I): 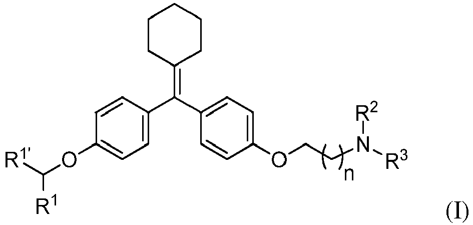 or salts thereof, wherein n, R¹, R^{1'}, R², and R³ are as defined below.

### BACKGROUND OF INVENTION

Cells respond to external signals by modifying their internal state and their environment. In multicellular organisms in particular, cellular differentiation and intra-cellular signaling are essential for the coordinated development of the organism. Revealing and understanding the spatiotemporal dynamics of these complex interaction networks is a major goal in biology.

While some of the most important players of these networks have been identified, much less is known of the quantitative rules that govern their interactions with one another and with other cellular components. Investigating these interactions requires the development of means to control or interfere spatially and temporally with these processes.

To address these issues, various approaches have been introduced to control proteins activity. One of these strategies directly acts at the protein level: the protein of interest is first inactivated and the activity of the protein is then restored with an appropriate stimulus in a controlled manner.

Photoactivation methods have proved particularly attractive for such control of protein's activity restoration due to their fast spatio-temporal dynamics.

For that purpose, genetically encoded photoactivable proteins were designed to intrinsically bear a non-photoactive site which can be activated by an inducer released upon photo-activation of a small lipophilic caged precursor. This method has been successfully implemented to photo-control gene expression in eukaryotic systems with one- and two-photon excitation.

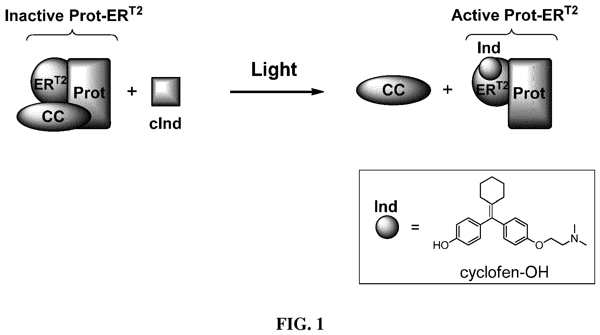

Especially, the Applicant has developed such a system to photocontrol protein activity (Sinha et al., ChemBioChem, 2010, 11, 653-663). In this system, a steroid-related inducer was used since various proteins, such as for example Engrailed, Otx2, Gal4, p53, kinases such as Raf-1, Cre or Flp recombinases, fused to a steroid receptor were shown to be activated by binding of an appropriate inducer such as cyclofen-OH (Figure 1).

As represented on Figure 1, in absence of this appropriate inducer (Ind), the steroid receptor (ER^{T2}) forms a cytoplasmic assembly with a chaperone complex (cc), inactivating the fusion protein (Prot). The function of the protein is restored in the presence of the inducer which binds to the receptor and disrupts the complex. The photocontrol of the activity of the targeted protein is performed by fusing it to the extensively used modified estrogen receptor ligand-binding domain (ER^{T2}) specific for the non-endogenous 4-hydroxy-cyclofen inducer, also called cyclofen-OH.

The Applicant evidenced that photo-releasing cyclofen-OH from a caged precursor (cInd) is an efficient strategy to restore the function of a protein fused to the ER^{T2} receptor. The dynamical effects of this system were investigated in cultured cells and in live zebrafish embryos. This method is compatible with a wide variety of proteins and may open up opportunities for the local spatio-temporal investigation of developmental pathways, the identification of stem cells and the study of cancer in a live organism.

In above-referred study (Sinha et al., ChemBioChem, 2010, 11, 653-663), the Applicant used 3 caged precursors (cInd, c'Ind and c"Ind) obtained through the following route of synthesis (Scheme 1):

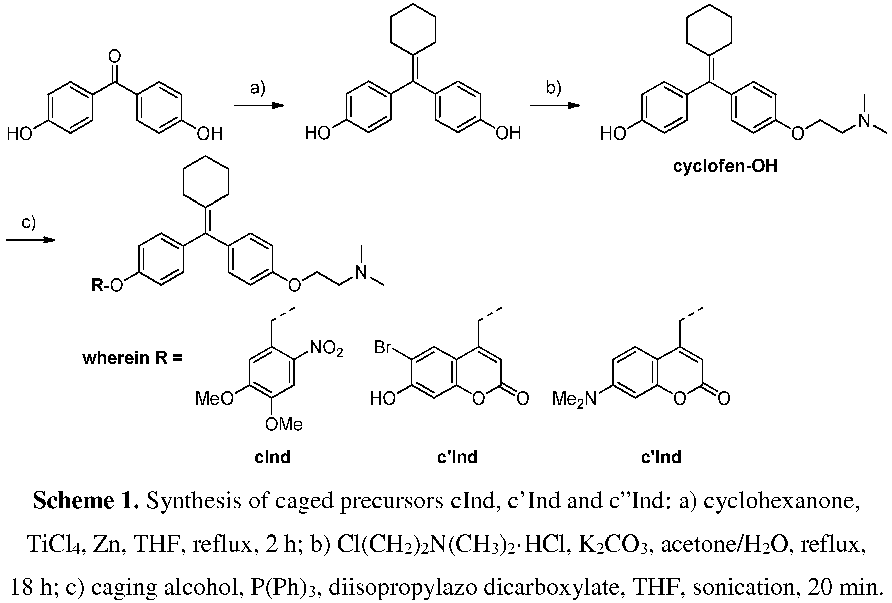

In this route of synthesis, a first step of McMurry coupling enables to introduce the cyclohexyl ring on 4,4'-dihydroxybenzophenone. Cyclofen-OH is then obtained by phenol monosubstitution. Caging of cyclofen-OH is then performed by a Mitsunobu reaction enabling to introduce the caging moiety.

Above route of synthesis presents the drawback to be not very reproducible.

Besides, the overall yields are respectively of 22%, 9.6% and 11% for cInd, c'Ind and c"Ind. These low yields are not compatible with synthesis at large scales.

Moreover, the caged precursor is obtained from the inducer (cyclofen-OH). A careful purification of the caged precursor is thus required so that the amount of remaining inducer in the final product be lower than 2%. Otherwise, the inducer present as an impurity will directly activate the protein, without control by photo-activation. In order to ensure such a low content of cyclofen-OH in the final product, a HPLC purification was needed. This is also not compatible with a scale-up process.

Above route of synthesis is thus not suitable for providing large amounts of caged precursors, which are required when dealing with demanded applications in animal models such as mice.

There is thus a need to provide a new process of synthesis of caged precursors of cyclofen-OH and derivatives thereof.

Patent application WO2008/030771 discloses modulators of estrogen receptor for therapeutic uses. Compounds of WO2008/030771 are cyclic alkylidene derivatives whose general formula encompasses cyclofen-OH. WO2008/030771 discloses three routes of synthesis, especially the route of synthesis of Scheme 2 below, leading to compound (8) via intermediate (7) (example 3 of WO2008/030771):

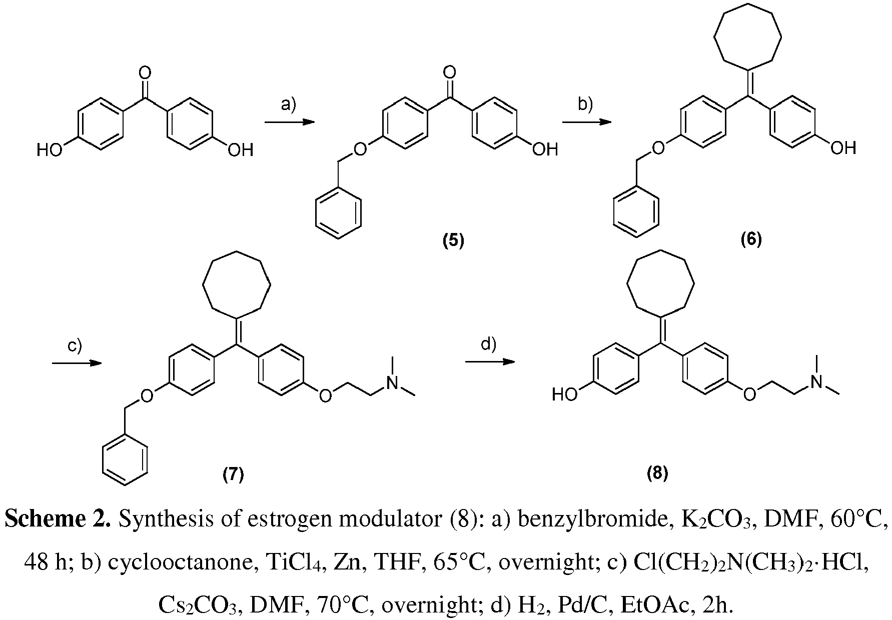

In this route of synthesis, a first step of phenol monoprotection is followed by a McMurry coupling to introduce the cycloalkyl ring. The second phenol is then substituted by alkylation and the final compound is released after deprotection.

With this route of synthesis, the overall yield for protected intermediate (7) is of 22%. As mentioned above with the other route of synthesis, such low yield is not suitable for scaling-up the process.

There is thus a need to provide an improved process of manufacturing of caged precursors of cyclofen-OH and derivatives thereof, being reproducible, providing an overall higher yield, not requiring sensible purification steps and being suitable for scale-up. The process of the present invention has such properties, as evidenced in the example part.

### DEFINITIONS

In the present invention, the following terms have the following meanings:
- "**alkoxy**" refers to any group -O-alkyl, wherein alkyl is as herein defined. Suitable alkoxy groups include for example methoxy, ethoxy, n-propoxy, isopropoxy, n-butoxy, t-butoxy, sec-butoxy, and n-pentoxy.
- "**alkyl**" refers to a hydrocarbyl radical of formula CₙH₂ₙ₊₁ wherein n is a number greater than or equal to 1. Generally, alkyl groups of this invention comprise from 1 to 12 carbon atoms, preferably from 1 to 6 carbon atoms. Alkyl groups may be linear or branched and may be substituted as indicated herein. Suitable alkyl groups include methyl, ethyl, propyl (n-propyl, i-propyl, n-butyl), butyl (i-butyl, s-butyl and t-butyl), pentyl and its isomers (e.g. n-pentyl, iso-pentyl), and hexyl and its isomers (e.g. n-hexyl, iso-hexyl).
- "**alkylamino**" refers to a group of formula -NH(alkyl), wherein alkyl is as herein defined.
- "**alkyloxyaryl**" refers to any group -aryl-O-alkyl, wherein aryl and alkyl are as herein defined.
- "**alkyloxycarbonylalkyloxy**" refers to any group -O-alkyl-(CO)-O-alkyl, wherein alkyl is as herein defined.
- "**amino**" refers to the -NH₂ group.
- "**aryl**" refers to a polyunsaturated, aromatic hydrocarbyl group having a single ring (i.e. phenyl) or multiple aromatic rings fused together (e.g. naphtyl) or linked covalently, typically containing 5 to 12 atoms; preferably 6 to 10, wherein at least one ring is aromatic. The aromatic ring may optionally include one to two additional rings (either cycloalkyl, heterocyclyl or heteroaryl) fused thereto. Aryl is also intended to include the partially hydrogenated derivatives of the carbocyclic systems enumerated herein. Non-limiting examples of aryl comprise phenyl, biphenylyl, biphenylenyl, 5- or 6- tetralinyl, naphthalen-1- or -2-yl, 4-, 5-, 6 or 7-indenyl, 1- 2-, 3-, 4- or 5-acenaphtylenyl, 3-, 4- or 5-acenaphtenyl, 1- or 2-pentalenyl, 4- or 5-indanyl, 5-, 6- , 7- or 8-tetrahydronaphthyl, 1,2,3,4-tetrahydronaphthyl, 1,4-dihydronaphthyl, 1-, 2-, 3-, 4- or 5-pyrenyl.
- "**arylethynyl**" refers to a group of formula -C=C-aryl, wherein aryl is as herein defined.
- "**arylvinylyl**" refers to a group of formula -CH=CH-aryl, wherein aryl is as herein defined.
- "**carbamimidoyl**" refers to the moiety -C(=NH)-NH₂.
- "**dialkylamino**" refers to a group of formula -N(alkyl)(alkyl), wherein alkyl is as herein defined.
- "**halo**" refers to fluoro, chloro, bromo, or iodo.
- "**haloalkoxy**" refers to any group alkoxy group substituted by one or more halo group. An example haloalkoxy group is -OCF₃.
- "**haloalkyl**" refers to any group alkyl group substituted by one or more halo group. Examples of preferred haloalkyl groups are CCl₃ and CBr₃.
- "**salt**" refers to the acid addition and base salts thereof. Suitable acid addition salts are formed from acids which form preferably non-toxic salts. Non-limiting examples include the acetate, trifluoroacetate, adipate, aspartate, benzoate, besylate, bicarbonate/carbonate, bisulphate/sulphate, borate, tetrafluoroborate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts. Suitable base salts are formed from bases which form preferably non-toxic salts. Non-limiting examples include the aluminium, arginine, benzathine, calcium, choline, diethylamine, diolamine, glycine, lysine, magnesium, meglumine, olamine, potassium, sodium, tromethamine, 2-(diethylamino)ethanol, ethanolamine, morpholine, 4- (2- hydroxyethyl)morpholine and zinc salts. Hemisalts of acids and bases may also be formed, for example, hemisulphate and hemicalcium salts. Preferred, pharmaceutically acceptable salts include hydrochloride/chloride, hydrobromide/bromide, bisulphate/sulphate, nitrate, citrate, and acetate.
- "**sulfonate**" refers to group of formula -OSO₂-R, wherein R represents alkyl, haloalkyl, optionally substituted aryl. Examples of preferred sulfonate groups are mesylate, triflate and tosylate.
- "**vinylcarboxy**" refers to a group of formula -CO-CH=CH₂.

### DETAILED DESCRIPTION

### Compounds

This invention relates to a process of manufacturing of caged cyclofen-OH and derivatives thereof, more specifically of compounds of formula (I): 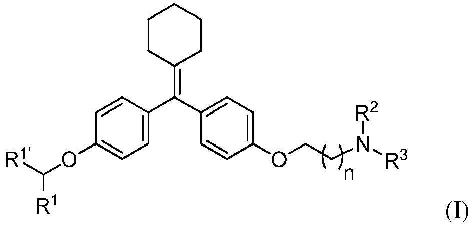 or salts thereof, wherein
R¹ represents Ar¹, -CO-Ph, -CH₂-(*o*-COR)Ph, -CH₂-(*o*-NO₂)Ph or -CO-CO-NR₂, wherein
   Ar¹ represents a phenyl or coumarin group, optionally substituted by one or more group selected from nitro, alkoxy, halo, hydroxyl, amino, alkylamino, dialkylamino, alkyloxycarbonylalkyloxy, alkyloxyaryl, aryl, arylvinylyl, arylethynyl and cyano;
   Ph represents a phenyl group, optionally substituted by one or more group selected from alkoxy, halo, hydroxyl, alkyl, haloalkyl, haloalkoxy, amino, alkylamino, dialkylamino, alkyloxycarbonylalkyloxy, alkyloxyaryl, aryl, arylvinylyl, arylethynyl and cyano; and
   R represents aryl, alkyl or vinylcarboxy; wherein the aryl group is optionally substituted by one or more group selected from alkoxy, halo, hydroxyl, alkyl, haloalkyl, haloalkoxy, amino, alkylamino, dialkylamino, alkyloxycarbonylalkyloxy, alkyloxyaryl, aryl, arylvinylyl, arylethynyl and cyano;
R^{1'} represents H, alkyl, haloalkyl, cyano or aryl;
R² and R³ represent each independently hydrogen, alkyl, carbomimidoyl; or R² and R³ form together with the nitrogen atom to which they are attached an heterocyclic ring preferably selected from pyrolidinyl, piperidinyl, N-methyl-piperazinyl, morpholinyl and pyrrolyl; and
n is an integer ranging from 1 to 6, preferably n is 1, 2, 3 or 4.

According to one embodiment, R¹ represents a moiety selected from: 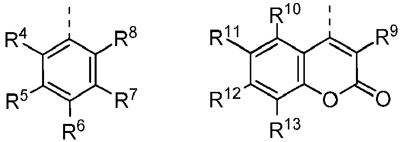 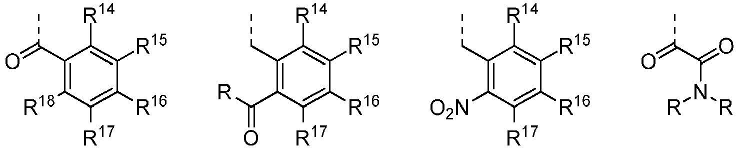 wherein
the dotted line represents the point of attachment to the rest of the molecule, R is as defined in formula (I); and
R⁴, R⁵, R⁶, R⁷, R⁸, R⁹, R¹⁰, R¹¹, R¹² and R¹³ represent each independently H, nitro, alkoxy, halo, hydroxyl, amino, alkylamino, dialkylamino, alkyloxycarbonylalkyloxy, alkyloxyaryl, aryl, arylvinylyl, arylethynyl or cyano; and
R¹⁴, R¹⁵, R¹⁶, R¹⁷ and R¹⁸ represent each independently H, alkoxy, halo, hydroxyl, alkyl, haloalkyl, haloalkoxy, amino, alkylamino, dialkylamino, alkyloxycarbonylalkyloxy, alkyloxyaryl, aryl, arylvinylyl, arylethynyl and cyano.

According to one embodiment, R^{1'} represents H or methyl, preferably R^{1'} represents H. According to one embodiment, R^{1'} represents haloalkyl, preferably -CCl₃ or -CBr₃. According to one embodiment, R^{1'} represents aryl, preferably phenyl.

According to one embodiment, n is equal to 1 or 2.

According to one embodiment, R² and R³ represent both methyl or R² and R³ represent both ethyl. According to one embodiment, R² represents H and R³ represents a carbomimidoyl group.

According to one embodiment, R² and R³ form together with the nitrogen atom to which they are attached a heterocyclic ring selected from pyrolidinyl, piperidinyl, N-methyl-piperazinyl, morpholinyl and pyrrolyl.

According to one embodiment, compounds of formula (I) are of formula (Ia) 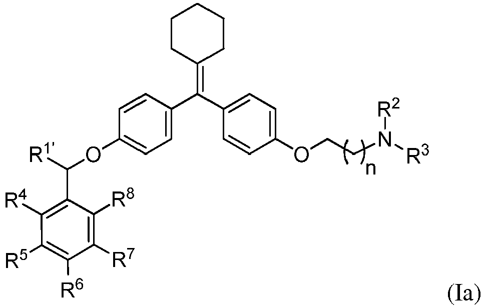 or salts thereof, wherein
n, R^{1'}, R² and R³ are as defined in formula (I), and
R⁴, R⁵, R⁶, R⁷ and R⁸ represent each independently H, nitro, alkoxy, halo, hydroxyl, amino, alkylamino, dialkylamino, alkyloxycarbonylalkyloxy, alkyloxyaryl, aryl, arylvinylyl, arylethynyl or cyano.

According to a specific embodiment, R⁴ is nitro. According to a specific embodiment, R⁴ is nitro and R⁸ is H. According to another specific embodiment, R⁴ and R⁸ are both nitro.

According to a specific embodiment, R⁵ is H.

According to a specific embodiment, R⁶ is an alkoxy, preferably methoxy. According to another specific embodiment, R⁶ is H.

According to a specific embodiment, R⁷ is alkoxy, alkyloxycarbonylalkyloxy or alkyloxyaryl, preferably R⁷ is methoxy, ethoxy, ethyloxycarbonylmethoxy or methoxyphenyl.

According to one embodiment, compounds of formula (I) are of formula (Ib) 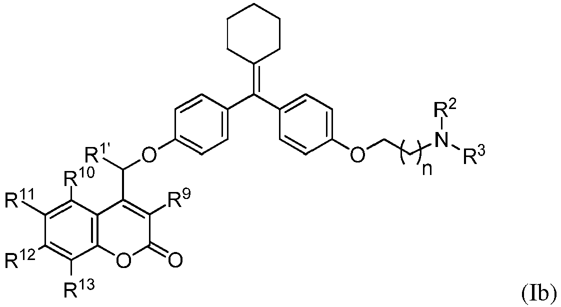 or salts thereof, wherein
n, R^{1'}, R² and R³ are as defined in formula (I), and
R⁹, R¹⁰, R¹¹, R¹² and R¹³ represent each independently H, nitro, alkoxy, halo, hydroxyl, amino, alkylamino, dialkylamino, alkyloxycarbonylalkyloxy, alkyloxyaryl, aryl, arylvinylyl, arylethynyl or cyano.

According to a specific embodiment, R⁹ is H. According to a specific embodiment, R¹⁰ is H. According to a specific embodiment, R¹³ is H. According to a specific embodiment, R⁹, R¹⁰ and R¹³ are H.

According to a specific embodiment, R¹¹ is H, alkoxy or halo, preferably R¹¹ is H, methoxy or bromo.

According to a specific embodiment, R¹² is alkoxy, hydroxyl or dialkylamino, preferably R¹² is methoxy, hydroxyl or dimethylamino.

In the present application, when it is referred to compounds of formula (I), it includes references to all subformulae thereof and to specific embodiments relative to substituents.

### Process

The process of the invention comprises a first step of McMurry coupling on 4,4'-dihydroxybenzophenone, enabling to introduce the cyclohexyl ring. The second step introduces the caging arylalkyl moiety by phenol monosubstitution. The second phenol group is then alkylated. This route of synthesis is summarized in Scheme 3 below:

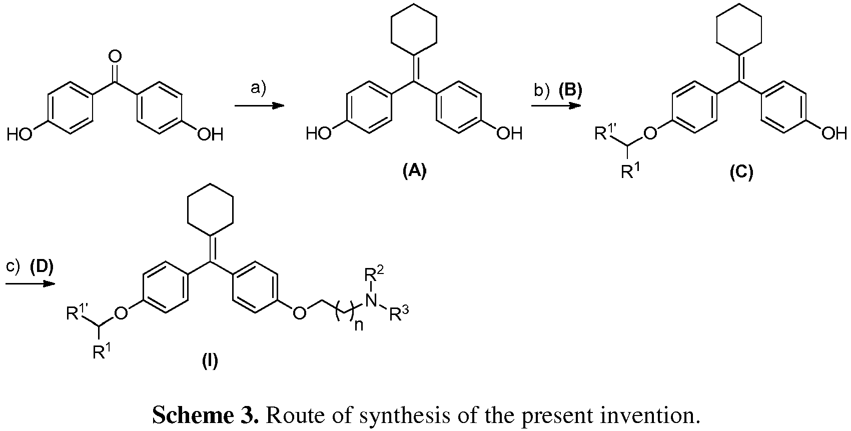

According to one embodiment, the process of the invention is a process of manufacturing a compound of formula (I) as defined above, comprising the following steps:
a) performing a McMurry coupling between 4,4'-dihydroxybenzophenone and cyclohexanone to form intermediate (A): 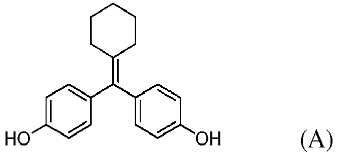
b) performing a phenol monosubstitution on intermediate (A) with caging halide (B) 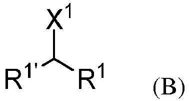 wherein R¹ and R^{1'} are as defined in formula (I) and X¹ represents halo, preferably X¹ represents Br or Cl, more preferably X¹ represents Br, to form intermediate (C) 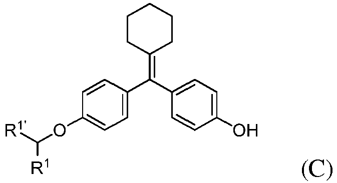 wherein R¹ and R^{1'} are as defined in formula (I),
   wherein step b) is performed in the dark, in a solvent in which intermediate (A) and caging halide (B) are soluble and in which intermediate (C) is not soluble; and
c) alkylating, in the dark, the remaining phenol of intermediate (C) with derivative (D) 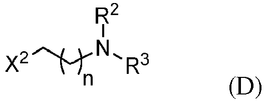 wherein n, R² and R³ are as defined in formula (I) and X² represents halo or sulfonate, preferably X² represents Cl, Br, I, mesylate, triflate or tosylate, more preferably X² represents Cl,
   to afford compound of formula (I).

### Step a)

According to one embodiment, cyclohexanone is used at a proportion ranging from 1 to 10 equivalents compared to 4,4'-dihydroxybenzophenone, preferably at a proportion of 3 to 6 equivalents, more preferably at a proportion of about 4.2 equivalents.

According to one embodiment, step a) is performed in presence of TiCl₄ and zinc. Advantageously, zinc is used under the form of powder. Advantageously, zinc is activated zinc.

According to one embodiment, TiCl₄ is used at a proportion ranging from 2 to 15 equivalents compared to 4,4'-dihydroxybenzophenone, preferably at a proportion of 4 to 10 equivalents, more preferably at a proportion of about 6.2 equivalents.

According to one embodiment, zinc is used at a proportion ranging from 2 to 30 equivalents compared to 4,4'-dihydroxybenzophenone, preferably at a proportion of 6 to 20 equivalents, more preferably at a proportion of about 14 equivalents.

According to one embodiment, the solvent used in step a) is selected from tetrahydrofuran (THF), 1,2-dimethoxyethane (DME), dioxane and tetrahydropyran; preferably the solvent is THF, more preferably dry THF.

According to one embodiment, the McMurry coupling of step a) is performed at a temperature ranging from 20 to 100°C, preferably at reflux of the solvent.

According to one embodiment, the McMurry coupling of step a) is performed for a duration ranging from 0.5 to 24 hours, preferably from 1 to 12 hours, more preferably for 2 hours.

Intermediate (A) obtained in step a) may be purified by flash chromatography or by precipitation. According to one embodiment, intermediate (A) is purified by flash chromatography on silica gel, using preferably a mixture cyclohexane/ethyl acetate as solvent. According to another embodiment, (A) is purified by precipitation and the solvent of precipitation is selected from dichloromethane, cyclohexane and a mixture ethyl acetate/cyclohexane.

### Step b)

According to one embodiment, caging halide (B) is used at a proportion ranging from 0.5 to 2 equivalents compared to intermediate (A), preferably at a proportion of 0.8 to 1.1 equivalents, more preferably at a proportion of about 0.95 equivalents.

According to one embodiment, the caging halide (B) is a bromide, i.e. X¹ represents Br. According to another embodiment, caging halide (B) is a chloride, i.e. X¹ represents Cl.

According to one embodiment, step b) is performed in presence of a base, preferably a base selected from K₂CO₃ and Cs₂CO₃, more preferably the base is K₂CO₃.

According to one embodiment, K₂CO₃ is used at a proportion ranging from 0.5 to 2 equivalents compared to intermediate (A), preferably at a proportion of 0.8 to 1.1 equivalents, more preferably at a proportion of about 0.95 equivalents.

According to one embodiment, NaI or KI may be added to perform step b) if the conversion rate is too low.

According to one embodiment, the solvent used in step b) is a solvent in which starting products are soluble and resulting compound is not soluble. In other words, the solvent used in step b) is a solvent in which intermediate (A) and caging halide (B) are soluble and in which intermediate (C) is not soluble. Such solvent enables to easily recover intermediate (C) as a precipitate by filtration. According to a preferred embodiment, the solvent used in step b) is a mixture of water and of a miscible organic solvent. Examples of organic solvents miscible in water are acetonitrile, acetone, dioxane, THF, alcohol (such as for example methanol, ethanol or butanol). According to one embodiment, the solvent used in step b) is selected from water, acetonitrile, acetone, dioxane, THF, alcohol (such as for example methanol, ethanol or butanol) or a mixture thereof; preferably the solvent is a mixture of acetonitrile and water, more preferably acetonitrile/water 2/1 (v/v).

According to one embodiment, step b) is performed at a temperature ranging from 0 to 100°C, preferably at room temperature.

According to one embodiment, step b) is performed for a duration ranging from 1 to 72 hours, preferably from 4 to 48 hours, more preferably for 16 to 24 hours.

Step b) is performed in the dark in order to avoid degradation of resulting intermediate (C) which is photosensitive.

Intermediate (C) obtained in step b) forms a precipitate in the conditions of the reaction. Advantageously, intermediate (C) does not need to be purified to be used in step c).

### Step c)

According to one embodiment, derivative (D) is used at a proportion ranging from 1 to 10 equivalents compared to intermediate (C), preferably at a proportion of 1 to 3 equivalents, more preferably at a proportion of about 2 equivalents.

According to one embodiment, step c) is performed in presence of a base, preferably a base selected from Cs₂CO₃ and K₂CO₃, more preferably the base is Cs₂CO₃.

According to one embodiment, Cs₂CO₃ is used at a proportion ranging from 2 to 11 equivalents compared to intermediate (C), preferably at a proportion of 2 to 4 equivalents, more preferably at a proportion of about 3 equivalents.

According to one embodiment, the solvent used in step c) is selected from acetone, acetonitrile and dimethylformamide; preferably the solvent is acetone.

According to one embodiment, step c) is performed at a temperature ranging from 20 to 155°C, preferably at reflux of the solvent.

According to one embodiment, step c) is performed for a duration ranging from 1 to 48 hours, preferably from 6 to 24 hours, more preferably for 18 hours.

Step c) is performed in the dark in order to avoid degradation of final compound of formula (I) which is photosensitive.

Final compound (I) obtained in step c) is purified by flash chromatography. According to one embodiment, compound (I) is purified by flash chromatography on silica gel, using preferably a mixture dichloromethane and methanol as solvent.

The process of the invention presents the advantage to have an overall yield of more than 30%, preferably of more than 40%, even more preferably of more than 45%. This overall yield is twofold compared to process disclosed in the prior art.

### BRIEF DESCRIPTION OF THE DRAWINGS

**Figure 1** is a scheme representing the strategy to photo-activate properly engineered proteins: a protein (Prot) fused to the ER^{T2} receptor (ER^{T2}) is inactivated by the assembly formed with a chaperone complex (cc). Upon photoactivation of a caged precursor (cInd), a non-endogenous inducer (Ind) is released, which binds to the ER^{T2} receptor and sets the protein fusion free from its assembly with the chaperone complex. The inducer (Ind) may be 4-hydroxy-cyclofen (also called cyclofen-OH).

### EXAMPLES

The present invention is further illustrated by the following examples.

### Abbreviations

ACN: acetonitrile
DMF: dimethylformamide
DMSO: dimethylsulfoxide
EtOAc: ethyl acetate
HPLC: high performance liquid chromatography
HRMS: high resolution mass spectroscopy
MS: mass spectroscopy
NMR: nuclear magnetic resonance
TFH: tetrahydrofurane

### Material

Commercially available reagents were used as obtained. Microanalyses were performed by the Service de Microanalyses de Gif sur Yvette. The ¹H and ¹³C NMR spectra were recorded at room temperature on Bruker AM 250, 300 or 400 spectrometers; chemical shifts are reported in ppm with protonated solvent as internal reference (¹H, CHCl₃ in CDCl₃ 7.26 ppm, CHD₂SOCD₃ in CD₃SOCD₃ 2.49 ppm, CHD₂COCD₃ in CD₃COCD₃ 2.05 ppm; ¹³C, ¹³CDCl₃ in CDCl₃ 77.0, ¹³CD₃SOCD₃ in CD₃SOCD₃ 39.7 ppm, ¹³CD₃COCD₃ in CD₃COCD₃ 29.9 ppm); coupling constants (J) are given in Hz. Mass spectra (chemical ionization and high resolution with NH₃ or CH₄) were performed by the Service de Spectrométrie de Masse de Chimie ParisTech or by the Service de Spectrométrie de Masse de l'ICOA (Orléans). Column chromatography was performed on silica gel 60 (0.040-0.063 nm; Merck). Analytical thin-layer chromatography (TLC) was conducted on Merck silica gel 60 F254 precoated plates.

### Example 1: Synthesis of intermediate (A)

### Step a): synthesis of 4-(Cyclohexylidene(4-hydroxyphenyl)methyl)phenol (A)

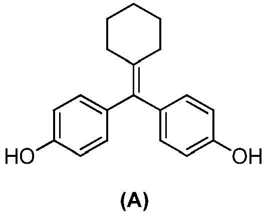

Titanium chloride (6.2 mL, 56 mmol) was added dropwise under argon to a stirred suspension of activated zinc powder (8.20 g, 126 mmol) in dry tetrahydrofuran (80 mL) at -10 °C. When the addition was complete, the mixture was warmed to room temperature and then refluxed for 2 h. A solution of 4,4'-dihydroxybenzophenone (2.0 g, 9 mmol) and cyclohexanone (4 mL, 38 mmol) in dry tetrahydrofuran (120 mL) was added to the cooled suspension of the titanium reagent at 0-5°C and the mixture was refluxed for 2 h. After being cooled to room temperature, the reaction mixture was quenched with 10% (w/v) aqueous potassium carbonate (30 mL), filtered and extracted with ethyl acetate. The organic layer was washed with brine, dried over MgSO₄, and concentrated *in vacuo.* Flash column chromatography (cyclohexane/ethyl acetate, 3:1, v/v) or precipitation in dichloromethane afforded (**A**) as a white powder respectively with (2.15 g, 85%) and (2.1 g, 81%); precipitation can also be carried out in cyclohexane or in a EtOAc/cyclohexane mixture. ¹H NMR (250 MHz, DMSO-d₆, δ): 9.28 (s, 2H), 6.82 (d, 4 H, J=8 Hz), 6.65 (d, 4H, J=8 Hz), 2.14 (m, 4 H), 1.52 (m, 6H); ¹³C NMR (100 MHz, DMSO-d₆, δ): 155.5 (2C), 136.2, 133.8, 133.6 (2C), 130.3 (4C), 114.6 (4C), 31.9 (2C), 28.1(2C), 26.3.

### Example 2: Synthesis of compound (1-1)

### Step b): synthesis of intermediate 4-((4-(4,5-dimethoxy-2-nitrobenzyloxy)phenyl) (cyclohexylidene)methyl)phenol (C-1)

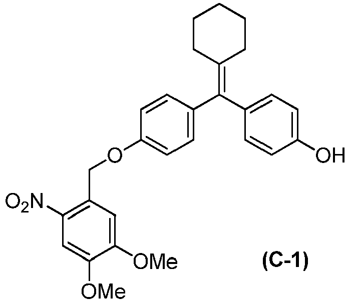

To a mixture of (**A**) (1 g, 3.5 mmol) and K₂CO₃ (470 mg, 3.4 mmol, 0.95 eq) in acetonitrile (ACN)/H₂O (60 mL, 2/1, v/v) was added portionwise 4,5-dimethoxy-2-nitrobenzyl bromide (**B-1**) (940 mg, 3.4 mmol, 0.95 eq) over a period of 1 h. The resulting mixture was stirred in the dark for 16 h. The precipitate was filtered, washed with ACN/H₂O and then dried *in vacuo* over P₂O₅. A white powder was obtained (1.4 g, **(A)/(C-1)/disubstituted by-product:** ε/96/4) and used without purification in the next step. ¹H NMR (ppm, 300 MHz, CDCl₃, δ): 7.77 (s, 1H), 7.35 (s, 1H), 7.04 (AA'XX', 2H, J= 9 Hz), 6.97 (AA'XX', 2H, J= 9 Hz), 6.91 (AA'XX', 2H, J= 9 Hz), 6.73 (AA'XX', 2H, J= 9 Hz), 5.48 (s, 2H), 4.68 (s, 1H), 3.97 (s, 3H), 3.95 (s, 3H), 2.23 (m, 4H), 1.57 (m, 6H). ¹³C NMR (75 MHz, DMSO-d₆, δ): 156.4, 155.9, 153.3, 148.0, 139.9, 137.1, 136.2, 133.7, 133.5, 130.7 (2C), 130.5 (2C), 122.5, 114.9 (2C), 114.5 (2C), 111.5, 108.4, 66.7, 56.3, 56.2, 32.1 (2C), 28.3 (2C), 26.4; MS (CI, NH3): m/z 493.30 (calculated average mass for [C₂₈H₂₉NO6 + NH₄]⁺: 493.23; HRMS (TOF MS ES+): m/z 476.2077 (calculated mass for [C₂₈H₂₉NO₆ + H]⁺: 476.2073.

### Step c): synthesis of 2-(4-((4-(4,5-Dimethoxy-2-nitrobenzyloxy)phenyl) cyclohexylidene)methyl)phenoxy)-N,N-dimethylethanamine (1-1)

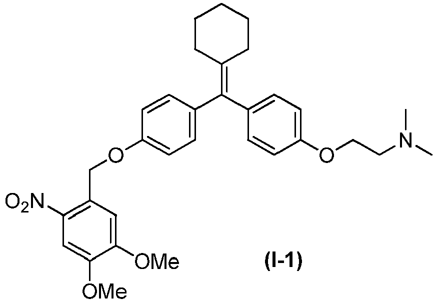

A solution of (**C-1**) (800 mg, 1.68 mmol) in acetone (50 mL) was treated with Cs₂CO₃ (1.64 g, 5 mmol, 3 eq.). 2-(Dimethylamino)ethylchlorid hydrochloride (**D-1**) (484 mg, 3.36 mmol, 2 eq.) was added portionwise over a period of 30 min and the resulting suspension was stirred at reflux temperature in the dark for 18 h. After being cooled to room temperature, the reaction mixture was filtered. The precipitate was washed with dichloromethane and the solvent was evaporated. Flash chromatography (CH₂Cl₂/MeOH: 9/1) afforded (**I-1**) (640 mg, 70% yield). ¹H NMR (400 MHz, CDCl₃, δ): 7.76 (s, 1H), 7.35 (s, 1 H), 7.04 (AA'XX', 2H, J=9 Hz), 7.00 (AA'XX', 2H, J=9 Hz), 6.90 (AA'XX', 2H, J=9 Hz), 6.82 (AA'XX', 2H, J=9 Hz), 5.47 (s, 2H), 4.03 (t, 2H, J= 6 Hz), 3.96 (s, 3 H), 3.94 (s, 3 H), 2.70 (t, 2H, J= 6 Hz), 2.32 (s, 6 H), 2.23 (m, 4H), 1.58 (m, 6H); ¹³C NMR (100 MHz, CDCl₃, δ): 157.0,156.2,153.8,147.7,138.9,138.5,136.8, 137.7, 133.2, 131.0 (2C), 130.7 (2C), 129.6, 114.3 (2C), 113.8 (2C), 109.4, 107.9, 67.0, 65.8, 58.3, 56.3, 56.3, 45.8 (2C), 32.4 (2C), 28.6 (2C), 26.8.

The overall yield for the synthesis of compound (I-1) from 4,4'-dihydroxybenzophenone is of 47%.

### Example 3: Synthesis of compound (1-2)

### Step b): synthesis of intermediate 4-((4-(6,7-dimethoxycoumarin)phenyl) (cyclohexylidene)methyl)phenol (C-2)

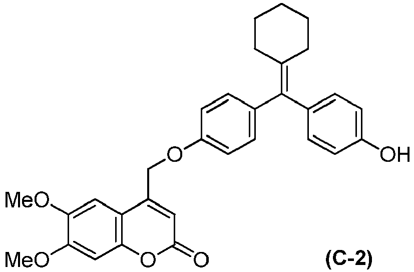

To a mixture of (**A**) (100 mg, 0.35 mmol) and K₂CO₃ (47 mg, 0.34 mmol, 0.95 eq) in ACN/H₂O (7 mL, 2/1, v/v) was added 4-bromomethyl-6,7-dimethoxycoumarin (**B-2**) (97 mg, 0.34 mmol, 0.95 eq). The resulting mixture was stirred in the dark for 24 h. The precipitate was filtered, washed with ACN/H₂O and then dried *in vacuo* over P₂O₅. A white powder was obtained (120 mg, **(A)/(C-2)/disubstituted by-product:** ε/90/10) and used without purification in the next step. ¹H NMR (ppm, 300 MHz, acetone-d6, δ): 7.30 (s, 1H), 7.08 (m, 4H), 6.99 (s, 1H), 6.93 (AA'XX', 2H, J= 9 Hz), 6.73 (AA'XX', 2H, J= 9 Hz), 6.45 (s, 1H), 5.41 (s, 2H), 3.97 (s, 3H), 3.987 (s, 3H), 2.24 (m, 4H), 1.59 (m, 6H). ¹³C NMR (75 MHz, CDCl3, δ): 160.3, 156.1, 155.9, 152.7, 151.6, 149.0, 145.8, 136.9, 136.4, 133.5, 132.9, 130.5 (2C), 130.3 (2C), 114.8 (2C), 114.5 (2C), 109.1, 108.7, 105.7, 100.3, 65.4, 56.1, 31.9 (2C), 28.1 (2C), 26.2; MS (ESI): m/z 499.5 (calculated mass for [C₃₁H₃₀O₆ + H]⁺: 499.2, m/z 521.5 (calculated mass for [C₃₁H₃₀O₆ + Na]⁺: 521.1).

### Step c): synthesis of 2-(4-((4-(4,5-Dimethoxycoumarin)phenyl) cyclohexylidene)methyl)phenoxy)-N,N-dimethylethanamine (I-2)

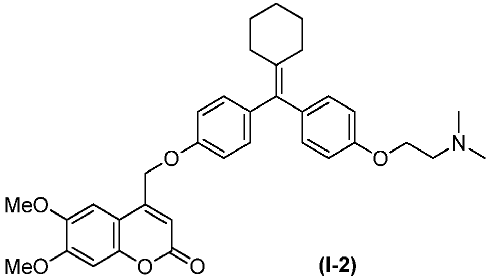

To a mixture of (**C-2**) (80 mg, 0.16 mmol) and Cs₂CO₃ (157 mg, 0.48 mmol, 3 eq.) in acetone (5 mL) was added 2-(dimethylamino)ethylchlorid hydrochloride (**D-1**) (46 mg, 0.32 mmol, 2 eq.). The resulting suspension was stirred at reflux temperature in the dark for 18 h. After being cooled to room temperature, the reaction mixture was filtered. The precipitate was washed with dichloromethane and the solvent was evaporated. Flash chromatography (CH₂Cl₂/MeOH: 9/1 v/v) afforded (**I-2**) (60 mg, 66% yield). ¹H NMR (300 MHz, CDCl₃, δ): 7.26 (s, 1H), 7.07 (m, 4 H), 7.01 (AA'XX', 2H, J=9 Hz), 6.95 (s, 1H), 6.85 (AA'XX', 2H, J=9 Hz), 6.44 (s, 1H), 5.38 (s, 2H), 4.09 (t, 2 H, J= 6 Hz), 3.93 (s, 3 H), 3.86 (s, 3 H), 2.73 (t, 2 H, J= 6 Hz), 2.30 (s, 6 H), 2.23 (m, 4H), 1.59 (m, 6H); ¹³C NMR (75 MHz, CDCl₃, δ): 161.1, 158.3, 157.5, 154.3, 152.0, 150.7, 147.4, 138.9, 137.7, 136.6, 134.7, 131.7 (2C), 131.6 (2C), 115.4 (2C), 114.8 (2C), 110.4, 110.4, 106.5, 101.1, 66.9, 66.7, 58.9, 56.8, 56.6, 46.1 (2C), 33.1 (2C), 29.4 (2C), 27.6. MS (ESI): m/z 570.5 (calculated mass for [C₃₅H₃₉O₆ + H]⁺: 570.2.

The overall yield for the synthesis of compound (I-1) from 4,4'-dihydroxybenzophenone is of 42%.

## Claims

1. A process of manufacturing a compound of formula (I) 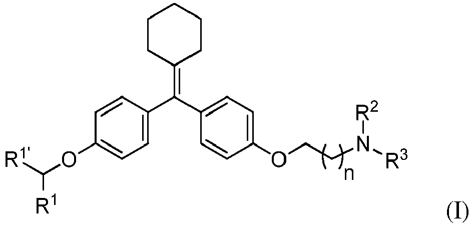 or a salt thereof, wherein
R¹ represents Ar¹, -CO-Ph, -CH₂-(*o*-COR)Ph, -CH₂-(*o*-NO₂)Ph or -CO-CO-NR₂, wherein
Ar¹ represents phenyl or coumarin groups, optionally substituted by a group selected from nitro, alkoxy, halo, hydroxyl, amino, alkylamino, dialkylamino, alkyloxycarbonylalkyloxy, alkyloxyaryl, aryl, arylvinylyl, arylethynyl, cyano;
Ph represents a phenyl group, optionally substituted by one or more group selected from alkoxy, halo, hydroxyl, alkyl, haloalkyl, haloalkoxy, amino, alkylamino, dialkylamino, alkyloxycarbonylalkyloxy, alkyloxyaryl, aryl, arylvinylyl, arylethynyl and cyano; and
R represents aryl, alkyl or vinylcarboxy; wherein the aryl group is optionally substituted by one or more group selected from alkoxy, halo, hydroxyl, alkyl, haloalkyl, haloalkoxy, amino, alkylamino, dialkylamino, alkyloxycarbonylalkyloxy, alkyloxyaryl, aryl, arylvinylyl, arylethynyl and cyano;
R^{1'} represents H, alkyl, haloalkyl, cyano, aryl;
R² and R³ represent each independently hydrogen, alkyl, carbamimidoyl; or R² and R³ form together with the nitrogen atom to which they are attached an heterocyclic ring, preferably selected from pyrrolidinyl, piperidinyl, N-methyl-piperazinyl, morpholinyl, pyrrolyl;
n is an integer ranging from 1 to 6, preferably n is 1, 2, 3 or 4, more preferably n is 1 or 2;
comprising the following steps:
a) performing a McMurry coupling between 4,4'-dihydroxybenzophenone and cyclohexanone to form intermediate (A) 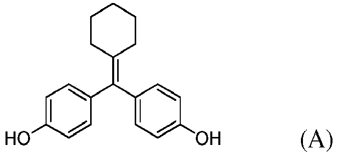
b) performing a phenol monosubstitution on intermediate (A) with caging halide (B) 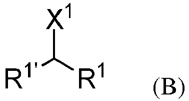 wherein R¹ and R^{1'} are as defined in formula (I) and X¹ represents halo, preferably X¹ represents Br or Cl, more preferably X¹ represents Br,
to form intermediate (C) 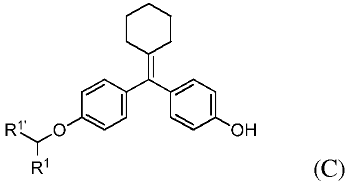 wherein R¹ and R^{1'} are as defined in formula (I),
wherein step b) is performed in the dark, in a solvent in which intermediate (A) and caging halide (B) are soluble and in which intermediate (C) is not soluble; and
c) alkylating in the dark the remaining phenol of intermediate (C) with derivative (D) 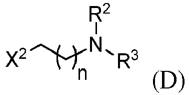 wherein n, R² and R³ are as defined in formula (I) and X² represents halo or sulfonate, preferably X² represents Cl, Br, I, mesylate, triflate or tosylate, more preferably X² represents Cl,
to afford compound of formula (I).

2. The process according to claim **1**, wherein step a) is performed in presence of TiCl₄ and zinc.

3. The process according to claim **1** or claim **2**, wherein cyclohexanone is used at a proportion ranging from 1 to 10 equivalents compared to 4,4'-dihydroxybenzophenone, preferably at a proportion of 3 to 6 equivalents, more preferably at a proportion of about 4.2 equivalents.

4. The process according to any one of claims **1** to **3**, wherein the solvent used in step a) is selected from tetrahydrofuran, 1,2-dimethoxyethane, dioxane and tetrahydropyran; preferably the solvent is tetrahydrofuran, more preferably dry tetrahydrofuran.

5. The process according to any one of claims **1** to **4**, wherein intermediate (A) is purified by precipitation and the solvent of precipitation is selected from dichloromethane, cyclohexane and a mixture ethyl acetate/cyclohexane.

6. The process according to any one of claims **1** to **5**, wherein step b) is performed in presence of a base, preferably a base selected from K₂CO₃ and Cs₂CO₃, more preferably the base is K₂CO₃.

7. The process according to any one of claims **1** to **6**, wherein caging halide (B) is used at a proportion ranging from 0.5 to 2 equivalents compared to intermediate (A), preferably at a proportion of 0.8 to 1.1 equivalents, more preferably at a proportion of about 0.95 equivalents.

8. The process according to any one of claims **1** to **7**, wherein the solvent used in step b) is selected from acetonitrile, water, acetone, dioxane, tetrahydrofuran, alcohol or a mixture thereof; preferably the solvent is a mixture of acetonitrile and water, more preferably acetonitrile/water 2/1 (v/v).

9. The process according to any one of claims **1** to **8**, wherein step c) is performed in presence of a base, preferably a base selected from Cs₂CO₃ and K₂CO₃, more preferably the base is Cs₂CO₃.

10. The process according to any one of claims **1** to **9**, wherein derivative (D) is used at a proportion ranging from 1 to 10 equivalents compared to intermediate (C), preferably at a proportion of 1 to 3 equivalents, more preferably at a proportion of about 2 equivalents.

11. The process according to any one of claims **1** to **10**, wherein the solvent used in step c) is selected from acetone, acetonitrile and dimethylformamide; preferably the solvent is acetone.

12. The process according to any one of claims **1** to **11**, wherein compound (I) is purified by flash chromatography.

## Patentansprüche

1. Ein Verfahren zur Herstellung von einer Verbindung der Formel (I) 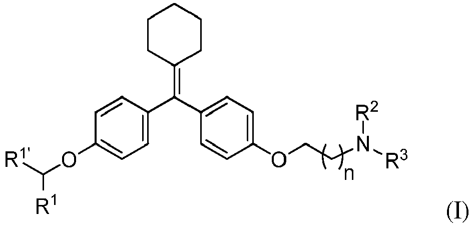 oder ein Salz davon, wobei
R¹ für Ar¹, -CO-Ph, -CH₂-(*o*-COR)Ph, -CH₂-(*o*-NO₂)Ph oder -CO-CO-NR₂ steht, wobei
Ar¹ für eine Phenyl- oder Cumaringruppe steht, die optional durch eine Gruppe substituiert ist, die aus Nitro, Alkoxy, Halo, Hydroxyl, Amino, Alkylamino, Dialkylamino, Alkyloxycarbonylalkyloxy, Alkyloxyaryl, Aryl, Arylvinylyl, Arylethinyl, Cyano ausgewählt ist;
Ph für eine Phenylgruppe steht, die optional durch eine oder mehrere Gruppen substituiert ist, die aus Alkoxy, Halo, Hydroxyl, Alkyl, Haloalkyl, Haloalkoxy, Amino, Alkylamino, Dialkylamino, Alkyloxycarbonylalkyloxy, Alkyloxyaryl, Aryl, Arylvinylyl, Arylethinyl und Cyano ausgewählt sind; und
R für Aryl, Alkyl oder Vinylcarboxy steht; wobei die Arylgruppe optional durch eine oder mehrere Gruppen substituiert ist, die aus Alkoxy, Halo, Hydroxyl, Alkyl, Haloalkyl, Haloalkoxy, Amino, Alkylamino, Dialkylamino, Alkyloxycarbonylalkyloxy, Alkyloxyaryl, Aryl, Arylvinylyl, Arylethinyl und Cyano ausgewählt sind;
R¹ für H, Alkyl, Haloalkyl, Cyano, Aryl steht;
R² und R³ jeweils unabhängig für Wasserstoff, Alkyl, Carbamimidoyl stehen; oder R² und R³ zusammen mit dem Stickstoffatom, an das sie angelagert sind, einen heterocyclischen Ring bilden, der vorzugsweise aus Pyrrolidinyl, Piperidinyl, N-Methylpiperazinyl, Morpholinyl, Pyrrolyl ausgewählt ist;
n eine ganze Zahl ist, die von 1 bis 6 reicht, vorzugsweise n 1, 2, 3 oder 4 ist, mehr bevorzugt n 1 oder 2 ist;
umfassend die folgenden Schritte:
a) Durchführen einer McMurry-Kopplung zwischen 4,4'-Dihydroxybenzophenon und Cyclohexanon, um Zwischenverbindung (A) zu bilden 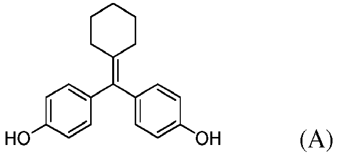
b) Durchführen einer Phenolmonosubstitution an Zwischenverbindung (A) mit einer Halogenid-Käfigverbindung (B) 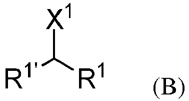 wobei R¹ und R^{1'} wie in Formel (I) definiert sind und X¹ für Halo steht, vorzugsweise X¹ für Br oder Cl steht, mehr bevorzugt X¹ für Br steht, um Zwischenverbindung (C) zu bilden 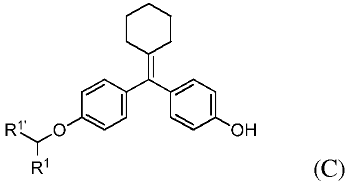 wobei R¹ und R^{1'} wie in Formel (I) definiert sind,
wobei Schritt b) im Dunkeln in einem Lösungsmittel, in dem Zwischenverbindung (A) und Halogenid-Käfigverbindung (B) löslich sind und in dem Zwischenverbindung (C) nicht löslich ist, durchgeführt wird; und
c) Alkylieren des restlichen Phenols von Zwischenverbindung (C) im Dunkeln mit Derivat (D) 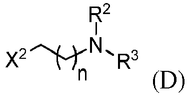 wobei n, R² und R³ wie in Formel (I) definiert sind und X² für Halo oder Sulfonat steht, vorzugsweise X² für Cl, Br, I, Mesylat, Triflat oder Tosylat steht, mehr bevorzugt X² für Cl steht,
um die Verbindung der Formel (I) zu liefern.

2. Das Verfahren nach Anspruch 1, wobei Schritt a) in Gegenwart von TiCl₄ und Zink durchgeführt wird.

3. Das Verfahren nach Anspruch **1** oder Anspruch **2**, wobei Cyclohexanon in einem Anteil, der von 1 bis 10 Äquivalente im Vergleich zu 4,4'-Dihydroxybenzophenon reicht, vorzugsweise in einem Anteil von 3 bis 6 Äquivalenten, mehr bevorzugt in einem Anteil von etwa 4,2 Äquivalenten verwendet wird.

4. Das Verfahren nach einem der Ansprüche **1** bis **3**, wobei das in Schritt a) verwendete Lösungsmittel aus Tetrahydrofuran, 1,2-Dimethoxyethan, Dioxan und Tetrahydropyran ausgewählt ist; vorzugsweise das Lösungsmittel Tetrahydrofuran, mehr bevorzugt trockenes Tetrahydrofuran ist.

5. Das Verfahren nach einem der Ansprüche **1** bis **4**, wobei Zwischenverbindung (A) mittels Ausfällung aufgereinigt wird und das Ausfällungslösungsmittel aus Dichlormethan, Cyclohexan und einem Ethylacetat/Cyclohexan-Gemisch ausgewählt ist.

6. Das Verfahren nach einem der Ansprüche **1** bis **5**, wobei Schritt b) in Gegenwart einer Base durchgeführt wird, vorzugsweise einer Base, die aus K₂CO₃ und Cs₂CO₃ ausgewählt ist, mehr bevorzugt die Base K₂CO₃ ist.

7. Das Verfahren nach einem der Ansprüche **1** bis **6**, wobei die Halogenid-Käfigverbindung (B) in einem Anteil, der von 0,5 bis 2 Äquivalente im Vergleich zu Zwischenverbindung (A) reicht, vorzugsweise in einem Anteil von 0,8 bis 1,1 Äquivalenten, mehr bevorzugt in einem Anteil von etwa 0,95 Äquivalenten verwendet wird.

8. Das Verfahren nach einem der Ansprüche **1** bis **7**, wobei das in Schritt b) verwendete Lösungsmittel aus Acetonitril, Wasser, Aceton, Dioxan, Tetrahydrofuran, Alkohol oder einem Gemisch davon ausgewählt ist; vorzugsweise das Lösungsmittel ein Gemisch von Acetonitril und Wasser, mehr bevorzugt Acetonitril:Wasser 2:1 (v/v) ist.

9. Das Verfahren nach einem der Ansprüche **1** bis **8**, wobei Schritt c) in Gegenwart einer Base durchgeführt wird, vorzugsweise einer Base, die aus Cs₂CO₃ und K₂CO₃ ausgewählt ist, vorzugsweise die Base Cs₂CO₃ ist.

10. Das Verfahren nach einem der Ansprüche **1** bis **9**, wobei Derivat (D) in einem Anteil, der von 1 bis 10 Äquivalente im Vergleich zu Zwischenverbindung (C) reicht, vorzugsweise in einem Anteil von 1 bis 3 Äquivalenten, mehr bevorzugt in einem Anteil von etwa 2 Äquivalenten verwendet wird.

11. Das Verfahren nach einem der Ansprüche **1** bis **10**, wobei das in Schritt c) verwendete Lösungsmittel aus Aceton, Acetonitril und Dimethylformamid ausgewählt ist; vorzugsweise das Lösungsmittel Aceton ist.

12. Das Verfahren nach einem der Ansprüche **1** bis **11**, wobei Verbindung (I) mittels Flash-Chromatographie aufgereinigt wird.

## Revendications

1. Un procédé de fabrication d'un composé de formule (I) 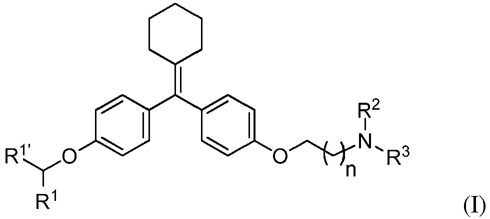 ou l'un de ses sels, dans lequel
R¹ représente Ar¹, -CO-Ph, -CH₂-(o-COR)Ph, -CH₂-(o-NO₂)Ph ou -CO-CO-NR₂, dans lequel
Ar¹ représente un groupe phényle ou coumarine, optionnellement substitué par un groupe choisi parmi nitro, alcoxyle, halo, hydroxyle, amino, alkylamino, dialkylamino, alkyloxycarbonylalkyloxyle, alkyloxyaryle, aryle, arylvinylyle, aryléthynyle, cyano ;
Ph représente un groupe phényle, éventuellement substitué par un ou plusieurs groupes choisis parmi alcoxyle, halo, hydroxyle, alkyle, haloalkyle, haloalcoxyle, amino, alkylamino, dialkylamino, alkyloxycarbonylalkyloxyle, alkyloxyaryle, aryle, arylvinylyle, aryléthynyle et cyano ; et
R représente un aryle, un alkyle ou un vinylcarboxy ; dans lequel le groupe aryle est optionnellement substitué par un ou plusieurs groupes choisis parmi alcoxyle, halo, hydroxyle, alkyle, haloalkyle, haloalcoxyle, amino, alkylamino, dialkylamino, alkyloxycarbonylalkyloxyle, alkyloxyaryle, aryle, arylvinylyle, aryléthynyle et cyano ;
R^{1'} représente H, alkyle, haloalkyle, cyano, aryle ;
R² et R³ représentent chacun indépendamment hydrogène, alkyle, carbamimidoyl ; ou R² et R³ forment ensemble avec l'atome d'azote auquel ils sont liés un cycle hétérocyclique, de préférence choisi parmi pyrrolidinyle, pipéridinyle, N-méthyl-pipérazinyle, morpholinyle, pyrrolyle;
n est un entier allant de 1 à 6, de préférence n est égal à 1, 2, 3 ou 4, plus préférablement n est égal à 1 ou 2 ;
comprenant les étapes suivantes :
a) effectuer un couplage de McMurry entre la 4,4'-dihydroxybenzophénone et la cyclohexanone pour former un intermédiaire (A) 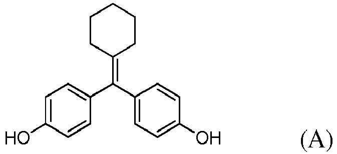
b) effectuer une monosubstitution d'un phénol sur l'intermédiaire (A) avec un halogénure de cage (B) 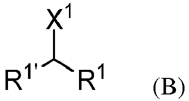 dans lequel R¹ et R^{1'} sont tels que définis dans la formule (I) et X¹ représente un halogène, de préférence X¹ représente Br ou Cl, plus préférablement X¹ représente Br,
pour former un intermédiaire (C) 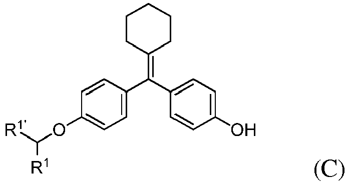 dans lequel R¹ et R^{1'} sont tels que définis dans la formule (I),
dans laquelle l'étape b) est réalisée dans l'obscurité, dans un solvant dans lequel l'intermédiaire (A) et l'halogénure de cage (B) sont solubles et dans lequel l'intermédiaire (C) n'est pas soluble ; et
c) alkyler dans l'obscurité le phénol restant de l'intermédiaire (C) avec un dérivé (D) 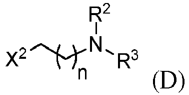 dans lequel n, R² et R³ sont tel que défini dans la formule (I) et X² représente un halogène ou un sulfonate, de préférence X² représente Cl, Br, I, mésylate, triflate ou tosylate, plus préférentiellement X² représente Cl,
pour donner le composé de formule (I).

2. Le procédé selon la revendication **1**, dans lequel l'étape a) est réalisée en présence de TiCl₄ et de zinc.

3. Le procédé selon la revendication **1** ou la revendication **2**, dans lequel la cyclohexanone est utilisée dans une proportion allant de 1 à 10 équivalents par rapport à la 4,4'-dihydroxybenzophénone, de préférence dans une proportion de 3 à 6 équivalents, plus préférentiellement dans une proportion d'environ 4,2 équivalents.

4. Le procédé selon l'une quelconque des revendications **1** à **3**, dans lequel le solvant utilisé dans l'étape a) est choisi parmi le tétrahydrofurane, le 1,2-diméthoxyéthane, le dioxane et le tétrahydropyrane ; de préférence le solvant est le tétrahydrofurane, plus préférentiellement le tétrahydrofurane sec.

5. Le procédé selon l'une quelconque des revendications **1** à **4**, dans lequel l'intermédiaire (A) est purifié par précipitation et le solvant de précipitation est choisi parmi le dichlorométhane, le cyclohexane et un mélange acétate d'éthyle/cyclohexane.

6. Le procédé selon l'une quelconque des revendications **1** à **5**, dans lequel l'étape b) est réalisée en présence d'une base, de préférence d'une base choisie parmi K₂CO₃ et Cs₂CO₃, plus préférentiellement la base est K₂CO₃.

7. Le procédé selon l'une quelconque des revendications **1** à **6**, dans lequel l'halogénure de cage (B) est utilisé dans une proportion allant de 0,5 à 2 équivalents par rapport à l'intermédiaire (A), de préférence dans une proportion de 0,8 à 1,1 équivalents, plus préférentiellement dans une proportion égale à d'environ 0,95 équivalents.

8. Le procédé selon l'une quelconque des revendications **1** à **7**, dans lequel le solvant utilisé dans l'étape b) est choisi parmi l'acétonitrile, l'eau, l'acétone, le dioxane, le tétrahydrofurane, l'alcool ou l'un de leurs mélanges ; de préférence le solvant est un mélange d'acétonitrile et d'eau, plus préférentiellement acétonitrile/eau 2/1 (v/v).

9. Le procédé selon l'une quelconque des revendications **1** à **8**, dans lequel l'étape c) est réalisée en présence d'une base, de préférence une base choisie parmi Cs₂CO₃ et K₂CO₃, plus préférentiellement la base est Cs₂CO₃.

10. Le procédé selon l'une quelconque des revendications **1** à **9**, dans lequel le dérivé (D) est utilisé dans une proportion allant de 1 à 10 équivalents par rapport à l'intermédiaire (C), de préférence dans une proportion de 1 à 3 équivalents, plus préférentiellement dans une proportion d'environ 2 équivalents.

11. Le procédé selon l'une quelconque des revendications **1** à **10**, dans lequel le solvant utilisé dans l'étape c) est choisi parmi l'acétone, l'acétonitrile et le diméthylformamide ; de préférence le solvant est l'acétone.

12. Le procédé selon l'une quelconque des revendications **1** à **11**, dans lequel le composé (I) est purifié par chromatographie flash.
